# EUROPÄISCHE PATENTSCHRIFT

(11) **EP 0 674 038 B1**
(45) Veröffentlichungstag und Bekanntmachung des Hinweises auf die Patenterteilung: **29.10.1997**
(21) Anmeldenummer: 95810196.6
(22) Anmeldetag: 22.03.1995
(51) Int. Cl.: D06M 13/355, C07D 265/22, D06P 1/642

(54) **Verwendung von 4H-3,1-Benzoxazin-4-on-Verbindungen zur Verbesserung der Lichtechtheit von Textilmaterialien**
Use of 4-H-3,1-benzoxazin-4-one compounds for improving the light fastness of textile materials
Utilisation de composés de 4-H-3,1-benzoxazine-4-one pour améliorer la solidité à la lumière de matériaux textiles

(30) Priorität: 26.03.1994 DE 4410539
(43) Veröffentlichungstag der Anmeldung: 27.09.1995
(73) Patentinhaber: Clariant Finance (BVI) Limited, Road Town, Tortola (VG)
(72) Erfinder: Mura, Jean-Luc, F-68170 Rixheim (FR)
(74) Vertreter: D'haemer, Jan Constant

(56) Entgegenhaltungen:
- EP-A- 0 068 327
- EP-A- 0 490 819
- CHEMICAL ABSTRACTS, vol. 120, no. 12, 1994 Columbus, Ohio, US; abstract no. 136958x,
- CHEMICAL ABSTRACTS, vol. 120, no. 10, 1994 Columbus, Ohio, US; abstract no. 109408m,

## Beschreibung

Die vorliegende Erfindung betrifft ein Verfahren zur Verbesserung der Lichtechtheit von Textilmaterialien, worin die Textilmaterialien mit einer wässrigen Flotte die 4H-3,1-Benzoxazin-4-on-Verbindungen enthält, behandelt werden.

Ferner betrifft die Erfindung die durch das Verfahren erhältlichen Textilien und deren Verwendung, sowie spezielle wäßrige Dispersionen, welche für die Durchführung des Verfahrens geeignet sind.

Solche Verbindungen sind z.B. aus DE-A-2556590, US-A-3,408,326 und EP-A-0 068 327 zumindest teilweise bekannt. In der DE-A-2 556 590 sind auch wäßrige Suspensionen beschrieben (im Rahmen der Herstellung, Beispiel 24). Aus EP-A-0 068 327 ist insbesondere bekannt, dass solche Verbindungen Polymerzusammensetzungen vor Ultraviolettlicht schützen. EP-A-0 068 327 beschreibt hauptsächlich den Schutz von Plastikartikeln oder transparenten organischen oder anorganischen Materialien, die der Sonne ausgesetzt sind. Der Schutz wird dadurch erreicht, dass die 4H-3,1-Benzoxazin-4-on-Verbindung in unreagiertem Zustand in das Polymer eines geformten Artikels inkorporiert wird oder indem ein geformter Artikel zuerst hergestellt und danach mit einem Ueberzug versehen oder imprägniert wird. Bei der Beschichtung wird vorzugsweise eine Lösung verwendet, welche ein zusätzliches Polymer enthält. Bei der Imprägnierung wird eine Lösung der 4H-3,1-Benzoxazin-4-on-Verbindung in einem organischen Lösungsmittel (z.B. Ketone, Ester, chlorierte Kohlenwasserstoffe und Amide) zubereitet und der Polymerartikel gegebenenfalls unter Erhitzen darin eingetaucht (S. 20, 2. Absatz). Obwohl auf S. 21, Zeile 8 von EP-A-0 068 327 Tuch/Stoff ("cloths") erwähnt wird, und auf Seite 17, Zeile 29 Fasern ("fibers"), wird sonst nirgends in dieser Patentschrift auf Textilien eingegangen, sondern es werden als zu schützende Polymerartikel hauptsächlich Filme, Platten oder hohle Artikel wie Rohre oder Behälter erwähnt.

[2,2'-Di-(4H-3,1-benzoxazin-4-on)]phenylen-Derivate werden neben zahlreichen anderen 4H-3,1-Benzoxazin-4-on-Verbindungen als geeignet beschrieben.

JP-A-5,195,367 offenbart einen UV-abschirmenden Polyester-Textilstoff hergestellt aus Filamenten, die durch Extrusion eines PET, das mit einem UV-Absorber, wie 2,2'-phenylen-bis-(3,1-oxazin-4-on) geblendet wurde, fabriziert wurden. Dieses Dokument offenbart keine wässrige Dispersion eines UV-Absorbers.

JP-A-5,230,346 offenbart ein Verfahren zur Spinnfärbung von Polyester-Fasern wobei der Schmelze Reaktionsprodukte von cyclischen Iminoestern, wie z.B. Benzoxazinonen, zugesetzt werden.

Es existiert keine Offenbarung von wässrigen Dispersionen eines UV-Absorbers.

EP-A-0,490,819 offenbart stabile wässrige Dispersionen einer Mischung von UV-Absorbern, die aber eine andere Struktur haben, als die erfindungsgemässen UV-Absorber.

Die UV-Absorber werden aus wäßriger Flotte nach einem Hochtemperatur-Ausziehverfahren aufgebracht.

Der vorliegenden Erfindung liegt nun die Aufgabe zugrunde Verbindungen in Form einer wässrigen Dispersion vorzusehen, die aufgrund ihres hervorragenden Ausziehvermögens zur Imprägnierung von Textilien im Ausziehverfahren geeignet sind.

Diese Aufgabe wird dadurch gelöst, dass man ein Verfahren zu Verbesserung der Lichtechtheit von Textilmaterialien, dadurch gekennzeichnet, dass man das Textilmaterial mit einer wässrigen Flotte, die eine wässrige Dispersion einer oder mehrerer Verbindungen der Formel I 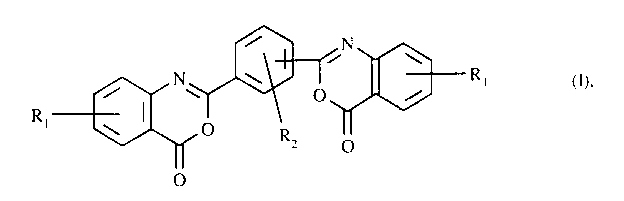 worin die
variable Bindung zwischen dem einen Benzoxazin-4-on-ring und dem Phenylen-Brückenglied in meta- oder para-Stellung zur Bindung des anderen Benzoxazin-4-on-rings am Phenylen-Brückenglied steht,
die beiden
- R₁: unabhängig voneinander Wasserstoff, Halogen, Nitro, C₁₋₂Alkyl, C₁₋₄Alkoxy, Carboxy, Sulfonsäure, C₁₋₃Alkylcarbonyl, Benzoyl oder C₁₋₄Alkylcarbonyl bedeuten und
- R₂: einen Substituenten aus der Reihe Wasserstoff, Chlor, Brom, Methyl oder C₁₋₂Alkoxy oder einen aus der Reihe Phenyl, Hydroxy, Nitro, Carboxy oder
Sulfonsäure bedeutet, enthält, nach dem Ausziehverfahren oder einem Klotz(Foulardier-) Verfahren behandelt, und fixiert, wobei das Fixieren bei der AusziehBehandlung oder durch nachträgliches Erhitzen erfolgt, bereitstellt.

Vorzugsweise bedeuten die beiden R, Wasserstoff, Chlor, Brom, Methyl oder C₁₋₂Alkoxy, insbesondere Wasserstoff.

R₂ bedeutet vorzugsweise Wasserstoff, Chlor, Nitro oder Methyl, insbesondere Wasserstoff.

Die Bindungen der Benzoxazin-4-on-ringe am Phenylen-Brückenglied befinden sich vorzugsweise in para-Stellung.

Prinzipiell können alle Textilmaterialien mit den Verbindungen der Formel I behandelt werden.

Diese Verbindungen werden wie die für das entsprechende Textilmaterial geeigneten Farbstoffe, vorzugsweise zusammen mit den Farbstoffen, beim Färben, appliziert. So werden für Fasern oder Fäden und die daraus hergestellten Textilien aus voll- oder halbsynthetischen, hydrophoben, hochmolekularen organischen Stoffen von wasserlöslichmachenden Gruppen freie Verbindungen der Formel I in fein dispergierter Form verwendet. Für Textilmaterialien aus natürlichen oder synthetischen Polyamiden und cellulosischem Material (nativ oder regeneriert) verwendet man vorzugsweise saure/anionische Verbindungen der Formel I.
Die Verbindungen der Formel I können auch im Gemisch mit anderen UV-Absorbern angewendet werden. Demzufolge sieht die vorliegende Erfindung auch wäßrige Dispersionen von Mischungen aus einer oder mehreren Verbindungen der Formel I wie oben definiert zusammen mit einem oder mehreren anderen UV-Absorbern vor. Solche andere UV-Absorber sind z.B. Verbindungen vom Typ 2,4-Dihydroxybenzophenon, 2,3,4-Trihydroxy-benzophenon, 2-Hydroxy-4-methoxybenzophenon, 2,2',4,4'-Tetrahydroxybenzophenon, 2,2'-Dihydroxy-4,4'-dimethoxybenzophenon, 2-Hydroxy-4-benzyloxybenzophenon, 2-(2H-Benzo-triazol-2-yl)-4,6-bis(1-methyl-1-phenyläthyl)phenol, 2-(2H-Benzotriazol-2-yl)-4-(1,1,3,3-tetra-methylbutyl)phenol, 2-(2H-Benzotriazol-2-yl)-4-(1,1 -dimethyläthyl)-6-( 1 -methylpropyl)-phenol, 2-(2H-benzotriazol-2-yl)-4,6-bis( 1-methylpropyl)phenol oder 2-(2'-Hydroxy-3'tert.butyl-5'-methyl)-5-chlorbenzotriazol.

Die Herstellung der Verbindungen der Formel I erfolgt auf die in der DE-A-25 56 590 oder US-A-3,408,326 beschriebene Weise.

Die Verbindungen der Formel I werden, wie schon oben erwähnt, wie die entsprechenden Farbstoffe auf die verschiedenen Substrate appliziert, wobei das Fixieren dabei ebenso erfolgt wie beim Färben üblich.

Ein grosser Vorteil dieser Verbindungen ist ihre hohe Sublimierechtheit. Auch die Nassechtheiten, insbesondere der von wasserlöslich machenden Gruppen freien Verbindungen, sind sehr gut. Hervorzuheben ist auch, dass die Verbindungen der Formel I im sichtbaren optischen Bereich keine Eigenfarbe aufweisen.

Die Verbindungen der Formel I werden im allgemeinen in Mengen von 0,05 bis 5, vorzugsweise 0,1 bis 3 und insbesondere 0,2 bis 3%, bezogen auf das Gewicht der mit ihnen zu behandelnden Textilmaterialien eingesetzt.

Gegenstand der Erfindung sind auch die durch das erfindungsgemäße Verfahren erhältlichen Textilien, sowie deren Verwendung zur sehr wirkungsvollen Abschirnung der die durch sie bedeckten Hautpartien ihres Trägers gegen die UV-Strahlung der Sonne.

In den folgenden Beispielen bedeuten die Teile Gewichtsteile und die Prozente Gewichtsprozente. I Volumenteil entspricht dem Volumen eines Gewichtsteiles Wasser (bei +4°C). Die Temperaturen sind in Celsiusgraden angegeben.

### BEISPIEL 1

163 Teile Isatoesäureanhydrid werden unter Rühren und wasserfreien Bedingungen in 1500 Teile Pyridin eingebracht, auf 60° erwärmt und portionenweise, ohne dass die Temperatur des Reaktionsgemisches über 80° ansteigt, mit 101,5 Teilen Terephthalsäuredichlorid versetzt. Das entstehende Kohlenstoffdioxid wird abgeleitet. Nach beendeter Terephthalsäuredichlorid-Zugabe kocht man 4 Stunden unter Rückflusskühlung, kühlt darauf auf Raumtemperatur, filtriert den unlöslichen Rückstand ab, wäscht ihn mit Methanol chlorfrei und trocknet ihn.

Das so erhaltene [2,2'-Di-(4H-3,1-benzoxazin-4-on)]-p-phenylen schmilzt bei 318°.

### BEISPIEL 2

Man verfährt wie im Beispiel 1 angegeben, setzt jedoch an Stelle von Terephthalsäuredichlorid die gleiche Menge Isophthalsäuredichlorid ein und erhält so einen in seiner lichtstabilisierenden Wirkung der gemäss Beispiel 1 erhaltenen Verbindungen ähnlichen Wirkstoff.

### BEISPIEL 3

5 Teile der gemäss Beispiel 1 erhaltenen Verbindung werden mit 2 Teilen eines Dispergators auf Basis Oleylalkohol/Aethylenoxid (Anlagerungsprodukt), 83 Teilen Wasser und 150 Teilen Glasperlen in einer geeigneten Dispergier-Apparatur 3 Stunden gemahlen. Dann ist die mittlere Teilchengrösse des Aktivstoffes unter 5 µm, die Glasperlen werden mit Hilfe eines Siebes von der Dispersion getrennt und mit 10 Teilen Wasser ausgewaschen. Die erhaltene Dispersion enthält 5% Aktivstoff.

### BEISPIEL 4

50 Teile eines Polyester-Trikotmaterials (Handelsname "Tersuisse") werden in 1000 Teile einer wässrigen Flotte, die

| | |
|---|---|
| 0,8 | Teile C.I. Disperse Yellow 42, |
| 0,135 | Teile C.I. Disperse Red 202, |
| 0,135 | Teile C.I. Disperse Red 86, |
| 5,0 | Teile der Dispersion gemäss Beispiel 3 und |
| 2,0 | Teile Ammonsulfat enthält, |

gegeben, der pH-Wert des Bades in einer HT-Färbeapparatur auf 4,5 gestellt, 5 Minuten bei 60° behandelt, danach in ca. 30 Minuten auf 130° erhitzt und 60 Minuten bei dieser Temperatur gefärbt. Nach Abkühlen auf 60°, wird die Färbung aus dem Bad genommen, gespült, 20 Minuten bei 80° auf übliche Weise mit einer alkalischen Lösung von Natriumhydrosulfit gereinigt, mit warmem Wasser gespült, mit Essigsäure neutralisiert, zentrifugiert und die Restfeuchte an der Luft getrocknet. Ein Teil der Färbung wird danach zusätzlich 60 Sekunden bei 210° behandelt.

Auf dieselbe Weise, jedoch ohne Lichtstabilisator, wurde eine zweite Färbung hergestellt und je eine Probe nach ISO 105-A02 "Grey Scale for Assessing Change in Colour", (im Atlasgerät Ci 35) belichtet. Die Lichtechtheiten (gemessen mit dem Minolta Chromameter 200) sind wie folgt:

| | | |
|---|---|---|
| Anzahl Belichtungszyklen | 2 Zyklen | 60 Sek, bei 210°C behandelt |
| ohne UV-Absorber, Noten | 2,5 | 2,4 |
| mit UV-Absorber, Noten | 3,5 | 3,4 |

(beste Note = 5) Die erhaltenen Noten zeigen die gute Wirkung des erfindungsgemäss verwendeten Stabilisators, sowie seine gute Sublimationsechtheit.

### BEISPIEL 5

15 Teile der gemäss Beispiel 1 erhaltenen Verbindung werden in einem geeigneten Gefäss mit 10 Teilen 2-(2'-Hydroxy-3'tert.butyl-5'-methyl)-5-chlorbenzotriazol, 15 Teilen handelsüblichem Kondensationsprodukt aus Ditolyläthersulfonat und Formaldehyd, 0,25 Teilen Fungizid, 2 Teilen eines Copolymers aus Äthylenoxid und Proplyenoxid und 57,75 Teilen entmineralisiertem Wasser gemischt und in einer Perlenmühle mit Glasperlen ca. 4 Std. gemahlen, bis die mittlere Partikelgrösse unter 1µm ist. Die erhaltene Dispersion wird von den Glasperlen abfiltriert.

### BEISPIEL 6

50 Teile eines Polyester-Trikotmaterials (Handelsname "Tersuisse") werden in 100 Teile einer wässrigen Flotte, die

| | |
|---|---|
| 0,8 | Teile C.I. Disperse Yellow 42, |
| 0,135 | Teile C.I. Disperse Red 202, |
| 0,135 | Teile C.I. Disperse Red 86, |
| 1,0 | Teile der Dispersion gemäss Beispiel 5 und |
| 2,0 | Teile Ammonsulfat enthält, |

gegeben, der pH-Wert des Bades in einer HT-Färbeapparatur auf 4,5 gestellt, 5 Minuten bei 60° behandelt, danach in ca. 30 Minuten auf 130° erhitzt und 30 Minuten bei dieser Temperatur gefärbt. Nach Abkühlen auf 60° wird die Färbung aus dem Bad genommen, gespült, reduktiv gereinigt, neutralisiert und an der Luft getrocknet. Ein Teil der Färbung wird zusätzlich noch bei verschiedenen Bedingungen fixiert.
Auf dieselbe Weise, jedoch ohne Lichtstabilisator, wurde eine zweite Färbung hergestellt, und je eine Probe nach ISO 105-B06 belichtet. Die Lichtechtheiten (gemessen mittels Graumassstab mit dem Minolta Chromameter 200) sind wie folgt:

| | | | | |
|---|---|---|---|---|
| Anzahl Belichtungszyklen: | 2 Zyklen | | | |
| | nicht | 60 Sek | 120 Sek | 60 Sek |
| | fixiert | 190° | 190° | 210° |
| Ohne UV-Absorber, Noten | 2,5 | 2,5 | 2,5 | 2,5 |
| mit UV-Absorber, Noten | 3,8 | 3,6 | 3,5 | 3,5 |

Die erhaltenen Noten zeigen die gute Wirkung der erfindungsgemäss verwendeten Mischung, sowie ihre gute Sublimationsechtheit.

## Patentansprüche

1. Verfahren zur Verbesserung der Lichtechtheit von Textilmaterialien, wobei man das Textilmaterial mit einer wässrigen Flotte, die eine wässrige Dispersion eines UV-Absorbers enthält, behandelt, dadurch gekennzeichnet, dass man das Textilmaterial mit einer wäßrigen Flotte, die eine wäßrige Dispersion einer oder mehrerer Verbindungen der Formel I 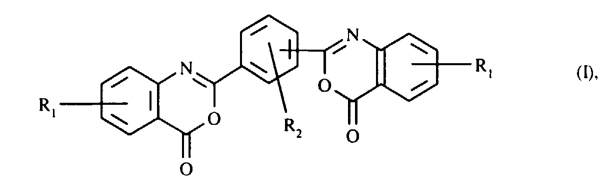 worin die
variable Bindung zwischen dem einen Benzoxazin-4-on-ring und dem Phenylen-Brückenglied in meta- oder para-Stellung zur Bindung des anderen Benzoxazin-4-on-rings am Phenylen-Brückenglied steht,
die beiden
R₁ unabhängig voneinander Wasserstoff, Halogen, Nitro, C₁₋₂Alkyl, C₁₋₄Alkoxy, Carboxy, Sulfonsäure, C₁₋₃Alkylcarbonyl, Benzoyl oder C₁₋₄Alkoxycarbonyl bedeuten und
R₂ einen Substituenten aus der Reihe Wasserstoff, Chlor, Brom, Methyl oder C₁₋₂Alkoxy oder einen aus der Reihe Phenyl, Hydroxy, Nitro, Carboxy oder
Sulfonsäure bedeutet, enthält, nach dem Ausziehverfahren oder einem Klotz(Foulardier-) Verfahren behandelt, und fixiert, wobei das Fixieren bei der AusziehBehandlung oder durch nachträgliches Erhitzen erfolgt.

2. Verfahren nach Anspruch 1, dadurch gekennzeichnet, dass die beiden R₁ unabhängig voneinander Wasserstoff, Chlor, Brom, Methyl oder C₁₋₂Alkoxy bedeuten.

3. Verfahren nach Anspruch 2, dadurch gekennzeichnet, dass die beiden R₁ Wasserstoff bedeuten.

4. Verfahren nach einem der vorhergehenden Ansprüche, dadurch gekennzeichnet, dass R₂ Wasserstoff, Chlor, Nitro oder Methyl bedeutet.

5. Verfahren nach Anspruch 4, dadurch gekennzeichnet, dass R₂ Wasserstoff bedeutet.

6. Verfahren nach einem der vorhergehenden Ansprüche, dadurch gekennzeichnet, dass die Bindung der Benzoxazin-4-on-ringe an das Phenylen-Brückenglied in para-Stellung ist.

7. Verfahren nach einem der vorhergehenden Ansprüche, dadurch gekennzeichnet, dass die wässrige Flotte 0,05 bis 5% einer oder mehrerer Verbindungen der Formel I wie in Anspruch 1 definiert, bezogen auf das Gewicht des zu behandelnden Textilmaterials enthält.

8. Wässrige Dispersion einer Mischung bestehend aus einer oder mehreren Verbindungen der Formel I, wie in Anspruch 1 definiert, zusammen mit einem oder mehreren anderen UV-Absorbern.

9. Wässrige Dispersion einer Mischung nach Anspruch 8, worin der andere UV-Absorber ausgewählt wird aus der Reihe der Verbindungen vom Typ 2,4-Dihydroxybenzophenon, 2,3,4-Trihydroxy-benzophenon, 2-Hydroxy-4-methoxybenzophenon, 2,2',4,4'-Tetrahydroxybenzophenon, 2,2'-Dihydroxy-4,4'-dimethoxybenzophenon, 2-Hydroxy-4-benzyloxybenzophenon, benzophenon, 2-(2H-Benzo-triazol-2-yl)-4,6-bis(1-methyl-1-phenyläthyl)phenol, 2-(2H-Benzotriazol-2-yl)-4-(1,1,3,3-tetramethylbutyl)phenol, 2-(2H-benzotriazol-2-yl)-4,6-bis( -dimethyläthyl)-6-(1-methylpropyl)-phenol, 2-(2H-benzotriazol-2-yl)-4,6-bis(1-methylpropyl)phenol und 2-(2'-Hydroxy-3'tert.butyl-5'-methyl)-5-chlorbenzotriazol.

10. Verfahren nach Anspruch 1, worin die wässrige Dispersion nach Anspruch 8 oder Anspruch 9 benutzt wird.

11. Textilmaterial, erhältlich durch ein Verfahren nach einem der Ansprüche 1 - 7 oder 10.

12. Textilmaterial gemäss Anspruch 11, dadurch gekennzeichnet, dass es aus Polyester besteht oder Polyester enthält.

13. Verwendung von Textilien nach Ansprüchen 11 oder 12 zum Schutz der durch sie bedeckten Hautpartien ihres Trägers vor den UV-Strahlen der Sonne.

## Claims

1. Process for improving the light fastness of textile materials in which the textile material is treated with an aqueous bath containing an aqueous dispersion of a UV-absorber, characterized in that the textile material is treated according to the exhaustion or to a padding (foulard) process with an aqueous bath containing an aqueous dispersion of one or more compounds of the formula I 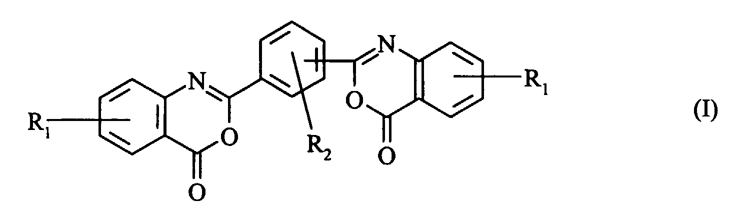 in which the variable link between the one benzoxazine-4-on ring and the phenylene bridging link is in position meta or para to the link of the other benzoxazine-4-on ring at the phenylene bridging link,
both R₁ independently from eachother are hydrogen, halogen, nitro, C₁₋₂alkyl, C₁₋₄alkoxy, carboxy, sulfonic acid, C₁₋₃alkylcarbonyl, benzoyl or C₁₋₄alkoxycarbonyl, and
R₂ is a substituent from the series hydrogen, chlorine, bromine, methyl or C₁₋₂alkoxy or from the series phenyl, hydroxy, nitro, carboxy or sulfonic acid
and the textile material is fixed in that the fixation is performed either in the exhaustion treatment or by heating afterwards.

2. Process according to claim 1, characterized in that both R₁ independently from eachother, are hydrogen, chlorine, bromine, methyl or C₁₋₂alkoxy.

3. Process according to claim 2, characterized in that both R₁ are hydrogen.

4. Process according to one of the preceding claims, characterized in that R₂ is hydrogen, chlorine, nitro or methyl.

5. Process according to claim 4, characterized in that R₂ is hydrogen.

6. Process according to one of the preceding claims, characterized in that the link of the benzoxazin-4-on rings to the phenylene bridging link is in position para.

7. Process according to one of the preceding claims, characterized in that the aqueous bath contains 0.05 to 5% based on the weight of the textile material to be treated, of one or more compounds of the formula I as defined in claim 1.

8. Aqueous dispersion of a mixture consisting of one or more compounds of the formula I as defined in claim 1, together with one or more other UV-absorbers.

9. Aqueous dispersion of a mixture according to claim 8 in which the other UV-absorber is selected from the series of compounds of the type 2,4-dihydroxybenzophenone, 2,3,4-trihydroxy-benzophenone, 2-hydroxy-4-methoxybenzophenone, 2,2',4,4'-tetrahydroxybenzophenone, 2,2'-dihydroxy-4,4'-dimethoxybenzophenone, 2-hydroxy-4-benzyloxybenzophenone, 2-(2H-benzotriazol-2-yl)-4,6-bis(1-methyl-1-phenylethyl)phenol, 2-(2H-benzotriazol-2-yl)-4-(1,1,3,3-tetramethylbutyl)phenol, 2-(2H-benzotriazol-2-yl)-4-(1,1-dimethylethyl)-6-(1-methylpropyl)-phenol, 2-(2H-benzotriazol-2-yl)-4,6-bis(1-methylpropyl)phenol or 2-(2'-hydroxy-3'-tert.butyl-5'-methyl)-5-chlorobenzotriazol.

10. Process according to claim 1 in which the aqueous dispersion according to claim 8 or 9 is used.

11. Textile material obtainable by a process according to one of the claims 1-7 or 10.

12. Textile material according to claim 11, characterized in that it consists of polyester or contains polyester.

13. Use of textile materials according to claims 11 or 12 for the protection of the skin portions of the wearer covered by the materials against UV radiation from the sun.

## Revendications

1. Procédé pour améliorer la solidité à la lumière de matériaux textiles par traitement des matériaux textiles avec un bain aqueux contenant une dispersion aqueuse d'un absorbant UV, caractérisé en ce que les matériaux textiles sont traités par un procédé par épuisement ou foulardage dans un bain aqueux contenant une dispersion aqueuse d'un ou plusieurs composés de formule (I) 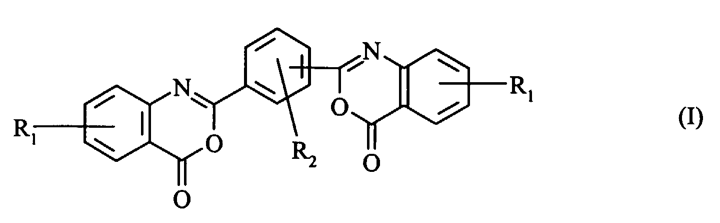 dans laquelle la liaison variable entre l'un des cycles benzoxazin-4-one et le pont phénylène se trouve en position méta ou para par rapport à l'autre cycle benzoxazin-4-one lié au pont phénylène,
les deux R₁, indépendamment l'un de l'autre, signifient hydrogène, halogène, nitro, C₁₋₂alkyle, C₁₋₄alkoxy, carboxy, sulfo, C₁₋₃alkylcarbonyl, benzoyl ou C₁₋₄alkoxycarbonyl, et
R₂ est un substituant de la série hydrogène, chlore, brome, méthyle ou C₁₋₂alkoxy ou un de la série phényle, hydroxy, nitro, carboxy ou sulfo,
et que les matériaux textiles sont fixés, le fixage étant effectué par épuisement ou par chauffage ultérieur.

2. Procédé d'après la revendication 1, caractérisé en ce que les deux R₁ signifient, indépendamment l'un de l'autre, hydrogène, chlore, brome, méthyle ou C₁₋₂alkoxy.

3. Procédé d'après la revendication 2, caractérisé en ce que les deux R₁ signifient hydrogène.

4. Procédé selon l'une des revendications précédentes, caractérisé en ce que R₂ signifie hydrogène, chlore, nitro ou méthyle.

5. Procédé selon la revendication 4, caractérisé en ce que R₂ signifie hydrogène.

6. Procédé selon l'une des revendications précédentes, caractérisé en ce que la liaison des cycles benzoxazin-4-one se trouve en position para du pont phénylène.

7. Procédé selon l'une des revendications précédentes, caractérisé en ce que le bain aqueux contient de 0,05 à 5% d'un ou plusieurs composés de formule (I) comme définie à la revendication 1, par rapport au poids du matériel textile à traiter.

8. Dispersion aqueuse d'un mélange consistant en un ou plusieurs composés de la formule (I) comme définie à la revendication 1, et un ou plusieurs autres absorbants UV.

9. Dispersion aqueuse d'un mélange selon la revendication 8, dans laquelle l'autre absorbant UV est choisi dans la série des composés du type 2,4-dihydroxybenzophénone, 2,3,4-trihydroxy-benzophénone, 2-hydroxy-4-méthoxybenzophénone, 2,2',4,4'-tétra-hydroxybenzophénone, 2,2'-dihydroxy-4,4'-diméthoxybenzophénone, 2-hydroxy-4-benzyloxybenzophénone, 2-(2H-benzotriazol-2-yl)-4,6-bis(1-méthyl-1-phényléthyl)-phénol, 2-(2H-benzotriazol-2-yl)-4-(1,1,3,3-tétra-méthylbutyl)phénol, 2-(2H-benzo-triazol-2-yl)-4-(1,1-diméthyléthyl)-6-(1-méthylpropyl)phénol, 2-(2H-benzotriazol-2-yl)-4,6-bis(1-méthylpropyl)phénol et 2-(2'-hydroxy-3'-tert.butyl-5'-méthyl)-5-chlorbenzo-triazole.

10. Procédé selon la revendication 1, dans lequel on utilise la dispersion aqueuse de la revendication 8 ou 9.

11. Matériel textile pouvant être obtenu par un procédé selon l'une des revendications 1-7 ou 10.

12. Matériel textile selon la revendication 11, caractérisé en ce qu'il est constitué de polyester ou en contient.

13. Utilisation des matériaux textiles selon les revendications 11 ou 12 pour la protection contre les rayons UV du soleil des parties de la peau du porteur couvertes pas ces matériaux textiles.
